# EUROPEAN PATENT SPECIFICATION

(11) **EP 4 032 514 B1**
(45) Date of publication and mention of the grant of the patent: **24.04.2024**
(21) Application number: 22159271.0
(22) Date of filing: 05.11.2015
(51) Int. Cl.: A61F 7/00, F04B 43/12, F04B 43/08, F04B 43/00, A61F 7/12

(54) **HEAT EXCHANGE SYSTEM FOR PATIENT TEMPERATURE CONTROL WITH EASY LOADING HIGH PERFORMANCE PERISTALTIC PUMP**
WÄRMEAUSTAUSCHSYSTEM ZUR PATIENTENTEMPERATURREGELUNG MIT LEICHT LADENDER HOCHLEISTUNGSFÄHIGER PERISTALTIKPUMPE
SYSTÈME D'ÉCHANGE DE CHALEUR DE RÉGULATION DE LA TEMPÉRATURE D'UN PATIENT DOTÉ D'UNE POMPE PÉRISTALTIQUE HAUTE PERFORMANCE À CHARGEMENT FACILE

(30) Priority: 06.11.2014 US 201414534718; 01.04.2015 US 201514676682
(43) Date of publication of application: 27.07.2022
(62) Divisional of application: 15857869.0
(73) Proprietor: ZOLL Circulation, Inc., San Jose, CA 95131 (US)
(72) Inventor: HENDRICKS, Austin Reid, Union City, 94587 (US); PAMICHEV, Christo Petrov, Cupertino, 95014 (US); GURUKULA, Venkata Vishnu, Mountain View, 94040 (US); DABROWIAK, Jeremy Thomas, Santa Clara, 95054 (US)
(74) Representative: Potter Clarkson

(56) References cited:
- EP-B1- 0 663 529
- DE-A1-102009 050 053
- US-A1- 2005 053 502
- US-A1- 2005 069 437
- US-A1- 2012 148 415

## Description

### TECHNICAL FIELD

The present application relates generally to heat exchange systems for patient temperature control with easy loading high performance peristaltic pumps.

### BACKGROUND

Patient temperature control systems have been introduced to prevent fever in patients in the neuro ICU due to suffering from sub-arachnoid hemorrhage or other neurologic malady such as stroke. Also, such systems have been used to induce mild or moderate hypothermia to improve the outcomes of patients suffering from such maladies as stroke, cardiac arrest, myocardial infarction, traumatic brain injury, and high intracranial pressure. Examples of intravascular heat exchange catheters are disclosed in U.S. Patent Nos. 7,914,564, 6, 416, 533, 6, 409, 747, 6,405,080, 6,393,320, 6,368,304, 6, 338, 727, 6,299,599, 6,290,717, 6,287,326, 6,165,207, 6,149,670, 6,146,411, 6,126,684, 6,306,161, 6,264,679, 6,231,594, 6,149,676, 6,149,673, 6,110,168, 5,989,238, 5,879,329, 5,837,003, 6,383,210, 6,379,378, 6,364,899, 6,325,818, 6,312,452, 6,261,312, 6,254,626, 6,251,130, 6,251,129, 6,245,095, 6,238,428, 6,235,048, 6,231,595, 6,224,624, 6,149,677, 6,096,068, 6,042,559, 8,888,729, and USPPs 2013/0178923, 2013/0079855, 2013/0079856, 2014/0094880, 2014/0094882, 2014/0094883.

External patient temperature control systems may be used. Such systems are disclosed in U.S. Patent Nos. 6,827,728, 6,818,012, 6,802,855, 6,799,063, 6,764,391, 6,692,518, 6,669,715, 6,660,027, 6,648,905, 6,645,232, 6,620,187, 6,461,379, 6,375,674, 6,197,045, and 6,188,930 (collectively, "the external pad patents").

In general, in all of the intravascular and external patient temperature control solutions, the temperature of the working fluid flowing through the catheter or pad is regulated by a heat exchange console based on feedback provided by the patient's actual body temperature, typically core body temperature as may be variously measured rectally, esophageally, tympanic ear temperature, blood temperature in, e.g., the vena cava, etc. The working fluid temperature is regulated by thermally coupling the working fluid to heating and/or cooling elements in the console. In many cases, the working fluid is forced in a closed fluid circuit path (including the console and the catheter or pad) by a peristaltic pump acting on tubing, e.g., pump tubing or IV tubing, in the fluid circuit path. DE 102009050053 describes a peristaltic pump comprising a head with pinch rollers which can be moved between a working position where at least one pinch roller clamps a pump hose against a hose bed and a release position providing free space between the pinch rollers and the hose bed. An eccentric drive and knurled wheel may be used to move the head between the working position and release position. US 2012/0148415 describes a pump rotor for a peristaltic hose pump and an actuating device by which the pump rotor can be actuated hydraulically and/or pneumatically. US 2005/0069437 describes a peristaltic pump having a pump head with rollers, and a backing plate which cooperates with the pump head to pinch a length of tubing between the rollers and the backing plate surface. US 2005/0053502 also describes a peristaltic pump. EP0663529 relates to a peristaltic pump assembly (10) for easy loading that includes a pump housing (24) having a curved surface.

### SUMMARY

As understood herein, peristaltic pumps typically include a rotor for revolving one or more rollers against an IV tube to force fluid through the tube by peristalsis, and an arcuate raceway against which the tube is urged by the rollers. The ease by which the tube can be loaded between the rollers and raceway competes with the performance of the pump: an easier to load pump typically has lower performance, whereas a higher performance pump (with higher pumping pressure and fluid flow) usually entails more complex loading of the tube. This is because in easy to load pumps, the raceway is typically movable away from the rollers to facilitate easily placing the tube between the rollers and raceway, but higher performance pumps require longer raceways (greater than 180 degrees of arc) that are generally not movable away from the pump, complicating the task of loading the tube (which for high performance applications is relatively thick and inflexible compared to low performance tubes) between the rollers and raceway.

The invention provides a pump assembly according to claim 1.

In examples, the pump assembly comprises a motor mount supporting the rotor motor, wherein the loading motor is coupled to the motor mount to move the motor mount and thereby move the rotor rotationally relative to the raceway such that motor mount is parallel to a plane of the bottom surface of the raceway when the rotor is in a pump position, and wherein the motor mount is angled relative to the plane of the bottom surface of the raceway when the rotor is in a tube load position.

In examples, the loading motor is configured to reciprocatingly drive a push rod to move the rotor between a pump position and a tube load position.

In examples, the push rod is the rack element of a rack-and-pinion gear, with the pinion portion being geared to a shaft rotatable by the motor.

In examples, the loading motor is configured to move the rotor translationally and rotationally relative to the raceway.

In examples the pump assembly comprises an operating element manipulable by a person or controller to energize the loading motor.

In examples the raceway has an inner surface extending through an arc of at least one hundred eighty degrees, the arc defining a midpoint, and the rotor faces the inner surface of the raceway, wherein the rotor is spaced from the midpoint a first distance in the pump position, and wherein the rotor is spaced from the midpoint a second distance greater than the first distance in the tube load position.

In examples, the inner surface of the raceway extends through an arc of at least one hundred eighty degrees.

In examples the pump assembly comprises: at least one drive roller on the rotor, the drive roller having a cylindrical outer surface, at least a portion of the outer surface being configured to urge against a tube disposed between the rotor and the raceway, the drive roller having no flanges with peripheries extending beyond the cylindrical outer surface; and at least one guide roller on the rotor, the guide roller having a cylindrical outer surface and top and bottom flanges defining respective peripheries extending beyond the cylindrical outer surface of the guide roller such that a tube disposed between the rotor and the raceway is receivable on the cylindrical outer surface of the guide roller between the flanges.

In examples the pump assembly comprises plural drive rollers and plural guide rollers on the rotor.

In examples, the raceway is an arcuate raceway having a concave inner surface extending through an arc of at least one hundred eighty degrees, the arc defining a midpoint; the rotor faces the inner surface of the raceway, the rotor being rotatable relative to the raceway, the rotor being mounted relative to the raceway between a pump position, wherein the rotor is spaced from the midpoint a first distance, and a tube load position, wherein the rotor is spaced from the midpoint a second distance greater than the first distance; and plural rollers arranged on the rotor to contact tubing disposed between the rotor and the raceway at least when the rotor is in the pump position, wherein the motor is prevented from stopping at at least one angular position in which at least one roller is in a predetermined location, and preferably, wherein the concave inner surface of the raceway extends through an arc of between one hundred eighty degrees and two hundred degrees.

In examples the rollers comprise: at least one drive roller on the rotor, the drive roller having a cylindrical outer surface, the entire outer surface being configured to urge against a tube disposed between the rotor and the raceway when the rotor is in the pump position and is rotated, the drive roller having no flanges with peripheries extending beyond the cylindrical outer surface; and at least one guide roller on the rotor, the guide roller having a cylindrical outer surface and top and bottom flanges defining respective peripheries extending beyond the cylindrical outer surface of the guide roller such that a tube disposed between the rotor and the raceway is received on the cylindrical outer surface of the guide roller between the flanges when the rotor is in the pump position and is rotated.

Also described herein is a heat exchange system for patient temperature control, the heat exchange system comprising a control system and a closed loop for working fluid, the closed loop comprising: a cassette engageable with the control system to exchange heat with the working fluid, a tube configured as a loop in fluid communication with the cassette, and an intravascular heat exchange catheter configured to be controlled by the control system to control the patient temperature wherein the control system comprises the pump assembly of any previous example, wherein the pump assembly is configured to engage the tube to urge the working fluid through the closed loop through peristalsis.

The details of the present application, both as to its structure and operation, can best be understood in reference to the accompanying drawings, in which like reference numerals refer to like parts, and in which:

### BRIEF DESCRIPTION OF THE DRAWINGS

Figure 1 is a schematic view of a non-limiting system,
Figure 2 is a perspective view of the pump with the rotor in the pump position;
Figure 3 is a top view of the pump with die rotor in the pump position;
Figure 4 is a perspective view of the pump with the rotor in the tube load position, illustrating a person manually loading the tube between the raceway and the rotor;
Figure 5 is a top view of the pump with the rotor in the tube load position;
Figures 6 and 7 are exploded perspective views from the bottom and top, respectively, of the pump, illustrating features of an example embodiment, with portions broken away in Figure 7;
Figures 8 and 9 are exploded side views respectively showing the relationship between the motor mount and the raceway in the tube load and pump positions of the rotor, with some portions broken away; and
Figure 10 is a perspective view of an embodiment of the invention, using a motor instead of a manually-operated handle with the rotor in the pump position, schematically illustrating the motor, gear, and operating button.

### DETAILED DESCRIPTION

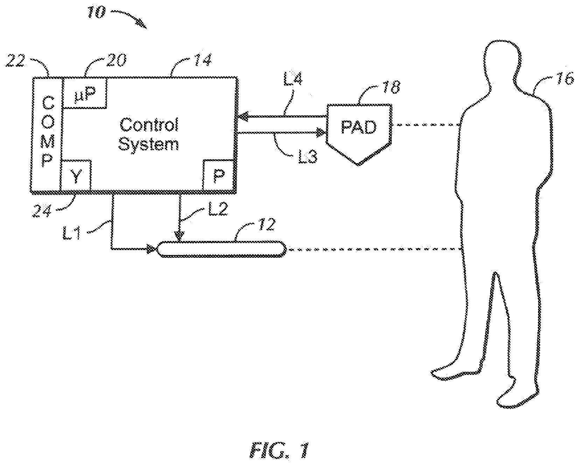

Referring initially to Figure 1, in accordance with present principles, a system 10 may include an intravascular heat exchange catheter 12 controlled by a control system 14 to control patient temperature, e.g., to prevent the patient 16 from becoming febrile or to induce therapeutic hypothermia in the patient 16. In the catheter, working fluid or coolant such as but not limited to saline circulates (typically under the influence of a pump "P" in the control system) in a closed loop from the control system 14, through a fluid supply line L1, through the catheter 12, and back to the system 14 through a fluid return line L2, such that no working fluid or coolant enters the body. While certain preferred catheters are disclosed herein, it is to be understood that other catheters can be used in accordance with present principles, including, without limitation, any of the catheters disclosed above or in the following U.S. patents: USPN 5,486,208, 5,837,003, 6,110,168, 6,149,673, 6,149,676, 6,231,594, 6,264,679, 6,306,161, 6,235,048, 6,238,428, 6,245,095, 6,251,129, 6,251,130, 6,254,626, 6,261,312, 6,312,452, 6,325,818, 6,409,747, 6,368,304, 6,338,727, 6,299,599, 6,287,326, 6,126,684, 7,211,106. The catheter 12 may be placed in the venous system, e.g., in the superior or inferior vena cava.

Instead of or in addition to the catheter 12, the system 10 may include one or more pads 18 that are positioned against the external skin of the patient 16 (only one pad 18 shown for clarity). The pad 18 may be, without limitation, any one of the pads disclosed in the external pad patents. The temperature of the pad 18 can be controlled by the control system 14 to exchange heat with the patient 16, including to induce therapeutic mild or moderate hypothermia in the patient in response to the patient presenting with, e.g., cardiac arrest, myocardial infarction, stroke, high intracranial pressure, traumatic brain injury, or other malady the effects of which can be ameliorated by hypothermia. The pad 18 may receive working fluid from the system 14 through a fluid supply line L3, and return working fluid to the system 14 through a fluid return line L4. The pump "P" may be a peristaltic pump which engages any one of the lines L1-L4, which are typically plastic IV lines, to urge working fluid through the lines through peristalsis.

The control system 14 may include one or more microprocessors 20 receiving target and patient temperatures as input and controlling, among other things, the pump "P" and a refrigerant compressor 22 and/or a bypass valve 24 that can be opened to permit refrigerant to bypass a condenser.

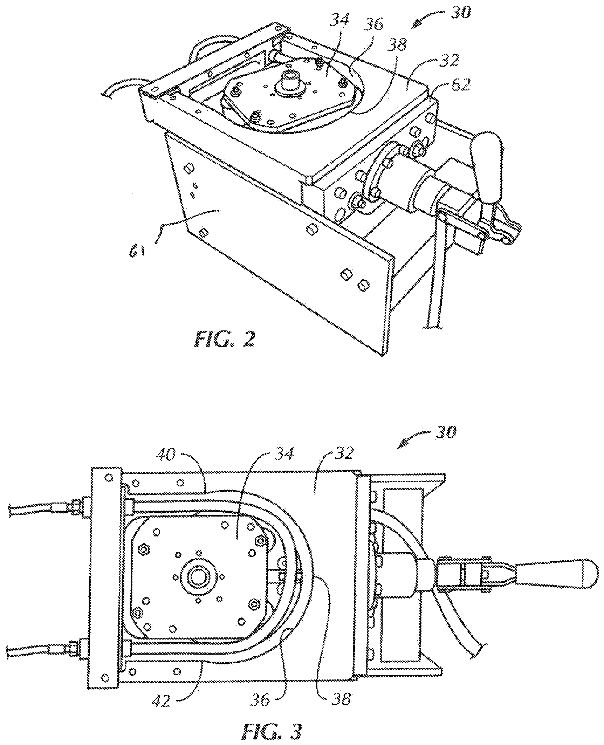

Turning now to Figures 2-5, an example of the pump "P" in Figure 1 is shown and generally designated 30. The pump 30 includes a rigid, preferably metal or hard plastic raceway 32 or channel and a rotor 34. The raceway 32 may be formed from one or more blocks of material as shown and has an inner arcuate surface 36 which may have a substantially constant radius of curvature. In some examples, the arcuate surface 36, which defines a midpoint 38 between its two ends 40, 42 (labeled in Figure 3), can extend through an arc of at least one hundred eighty degrees (180°) (e.g., where two drive rollers are used) and may extend through an arc of between one hundred eighty degrees (180°) and two hundred seventy degrees (270°). In the example shown, the arcuate surface 36 extends, from one end 40 to the other end 42, through an arc of greater than 200°. For example, the arc may be about 210° to 230°. In certain embodiments, the arcuate surface of a raceway may extend through an arc equal to 360° divided by n, where n is equal to the number of drive rollers mounted on or near a rotor which is rotated relative to the raceway.

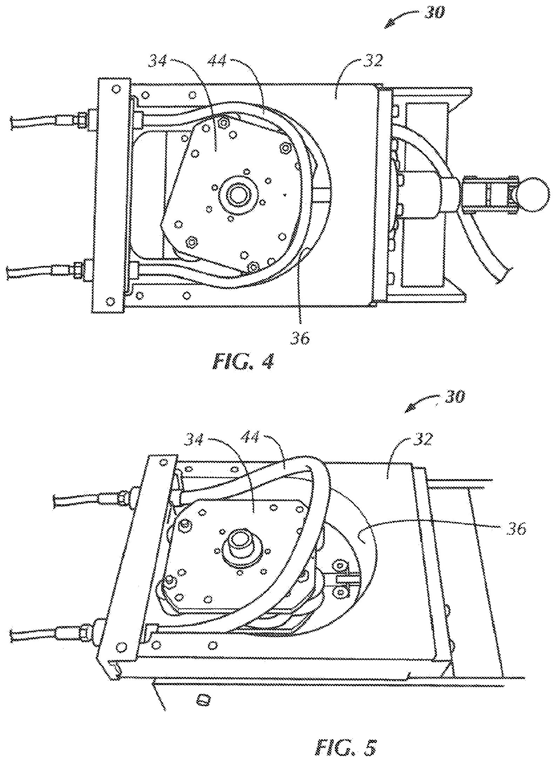

Covering more than 180° degrees of arc with the raceway may provide additional benefits, such as an extra margin against internal leakage. Furthermore, it is possible that covering more than 180° degrees of arc allows the tubing to open gradually after being compressed by a drive roller and thereby reduce the degree of pulsatility of the flow. This in turn can reduce the amount of unwanted movement experienced by the downstream tubing and catheter when subject to pulsating flow. A motor, described further below, rotates the rotor 34 relative to the raceway 32. As well, the rotor 34 is movable translationally and/or rotationally relative to the raceway 32 between a pump position (Figures 2, 3, 6, 7, and 9), in which the rotor 34 is spaced from the midpoint 38 of the inner surface 36 of the raceway 32 a first distance, and a tube load position (Figures 4, 5, and 8), in which the rotor 34 is spaced from the midpoint 38 a greater, second distance. As shown in Figures 2 and 3, in the pump position, rollers on the rotor 34 urge against a tube such as a pump tube or IV tube that is disposed between the rollers and the raceway 32. In the tube load position, the rotor 34 is sufficiently spaced from the raceway 32 to permit a tube 44 to be disposed between the raceway 32 and rotor 34 and to be removed therefrom, e.g., by hand. Example mechanisms for moving the rotor translationally and/or rotationally are discussed further below.

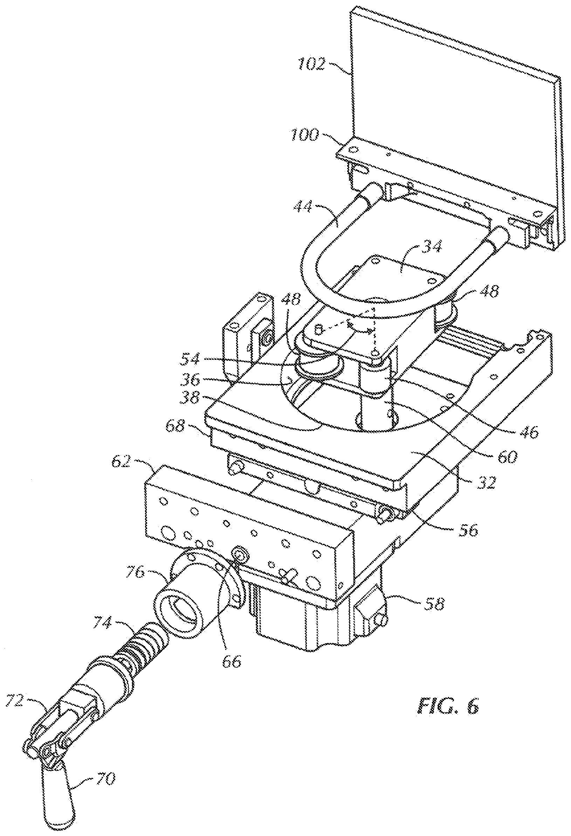

Indeed and now referring to Figures 6-9 for example structure, mounted on the rotor 34 are one or more rollers to urge against the tube 44 to pump fluid through the tube. In the example shown in Figure 6, the rotor 34 is defined in part by a rectilinear, non-square body, and on or near each corner of the body a roller is mounted, e.g., rotatably mounted to the rotor body. In the example, at one set of opposed corners on the body, drive rollers 46 are respectively mounted (only one drive roller shown in the perspective of Figure 6), whereas at the other set of opposed corners on the body, guide rollers 48 are respectively mounted. Thus, between the drive rollers 46 are guide rollers 48.

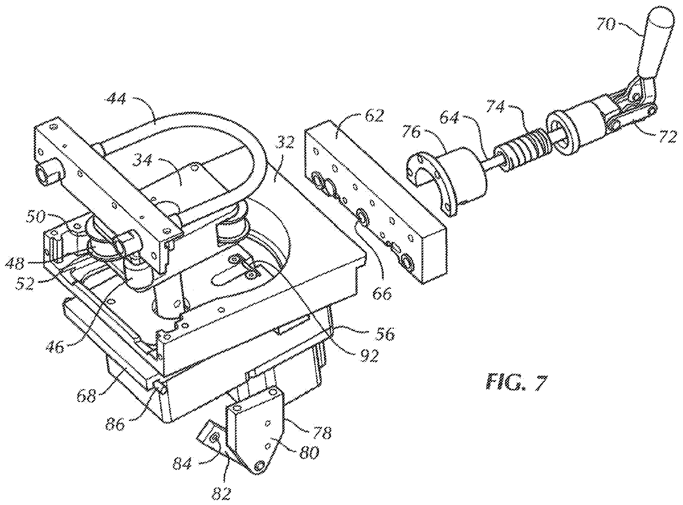

As shown in Figure 6, the drive roller 46 has a cylindrical or similar to cylindrical outer surface, and at least a portion of the outer surface is configured to urge against the tube 44. The outer surface of the example drive roller may be a single smooth cylinder and/or it may or may not have one or more flanges having peripheries extending beyond the cylindrical outer surface. In contrast, Figure 7 best shows that the guide roller 48 also has a cylindrical (or similar) outer surface but in addition includes top and/or bottom flanges 50, 52 defining respective peripheries extending beyond the cylindrical outer surface of the guide roller such that the tube 44 can be received on the cylindrical outer surface of the guide roller between the flanges 50. 52 when the rotor is in the pump position and is rotated. In the example shown, two and only two drive rollers 46 and two and only two guide rollers 48 are provided, but any number of drive and/or guide rollers may be utilized. In certain embodiments, the drive roller or guide roller may have a noncylindrical or partially cylindrical outer surface.

Also, in the example shown, owing to the non-square shape of the rotor 34 body, the angle 54 between the drive roller 46 and guide roller 48 at one of the ends of the rotor body, with a vertex on a point on the roller body (e.g., the midpoint), is not ninety degrees. Instead, in the example shown, the angle 54 may be, for example, fifty five degrees. The same angle obtains at the opposite end of the rotor body. However, in some embodiments the rotor body is square, in which case all rollers are separated from the adjacent rollers by ninety degrees.

A block-like motor mount 56 supports a motor 58 such as a small ac or de motor, in some embodiments, a stepper motor or other appropriate motor type. The motor 58 is coupled to the rotor 34 by an output shaft 60, with, in some embodiments, a reduction gear train (not shown) being meshed between the motor shaft and the output shaft 60.

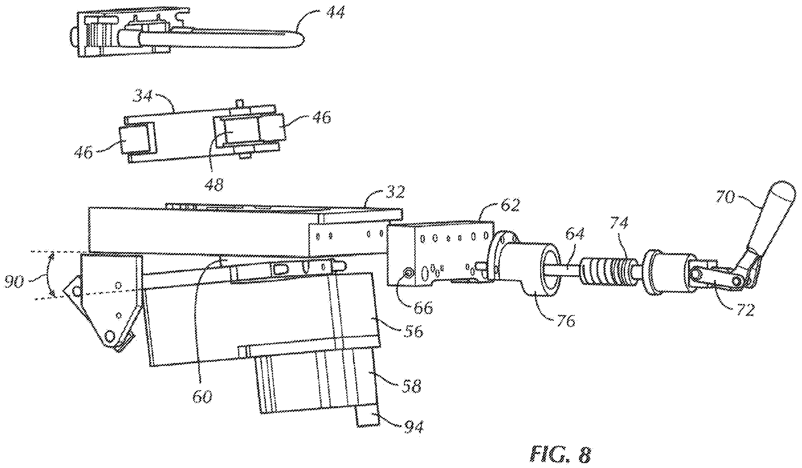

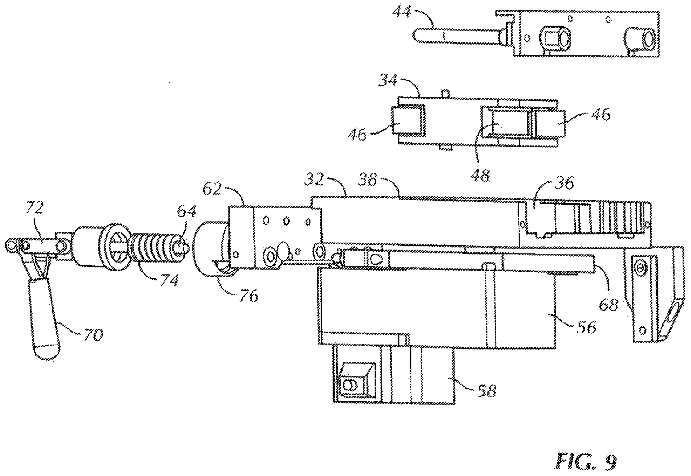

A positioning mechanism is coupled to the motor mount 56 and is manipulable by a person to move the motor mount 56 to thereby move the rotor 34 between the pump position and the tube load position. In a non-limiting example, referring briefly back to Figure 2, a base 61 stationarily holds the raceway 32, and a rectilinear rigid support block 62 (Figures 2 and 6-9) may be bolted or welded to the base 61 or made integrally therewith. A push rod 64 (Figures 7-9) extends through a hole 66 in the support block 62 to contact and/or be engaged with the motor mount 56 and/or with a motor plate 68 coupled to the motor mount 56. A handle 70 is coupled at a hinge mechanism 72 to the push rod 64. The handle 70 can be moved by hand to a substantially perpendicular orientation relative to the push rod 64 (Figures 6 and 9) to pull the push rod 64 and thus to move the motor mount 56 (and hence rotor 34) toward the inner surface of the raceway 32, thereby moving the rotor 34 to the pump position. The handle 70 can also be moved by hand down from the perpendicular orientation to the non-perpendicular orientation shown in Figures 7 and 8. This pushes the push rod 64 and thus moves the motor mount ,56:rotor 34 away from the pump position to the tube load position. One or more radial bearings 74, 76 may be provided as appropriate to radially support elements of the positioning mechanism.

Also and focusing on Figure 7, to support the motor mount 56 and attendant elements that move with it, two side brackets 78 may be provided on respective sides of the raceway 32 (only one bracket 78 shown in Figure 7). A vertical flange 80 of the side bracket 78 may be affixed to the raceway 32, e.g., by threaded fasteners or welding, and a swing arm 82 pivotably coupled to the vertical flange 80 and rotatably coupled to the motor mount 56 or other component that moves with the motor mount 56. In the example shown in Figure 7, a hole 84 is formed in the swing arm 82 and rotatably engages a pin 86 that is attached to and that extends radially away from the motor plate 68. Recall that the motor mount 56, motor plate 68, and rotor 34 move translationally together as unit.

Owing to the example positioning mechanism described above, as best shown in Figure 9 the motor mount 56 (with motor plate 68) is parallel to the raceway 32 when the rotor 34 is in the pump position. In contrast, as best shown in Figure 8, the motor mount 56 (with motor plate 68) is obliquely angled relative to the raceway 32 when the rotor 34 is in the tube load position. That is, an oblique angle 90 is established between, for example, the plane of the motor plate 68 and the plane defined by the bottom surface of the raceway 32 when the rotor 34 is in the tube load position. To further facilitate motion of the positioning mechanism when the handle 70 is moved, a hinge pin 92 (Figure 7) may be provided as part of the coupling between the push rod 64 and motor mount 56/motor plate 68.

Thus, the rotor 34 can move linearly relative to raceway 32. In the example shown, linear bearings are used, it being understood that equivalently a multi-bar linkage between the rotor 34 and raceway 32 can be used for pseudo-linear motion. In any case, in the tube position the rotor 34 is a sufficient distance (typically an inch or more) so that the tube 44 can be inserted freely between the rotor 34 and raceway 32 by a person. Then, when the rotor is moved to the pump position, at least the drive rollers 46 urge into the tube 44 sufficiently to stretch the tube 44 by an elongation of at least 3% and typically 3-15%. This elongation advantageously ensures that slack does not build up in the tubing as it wears and stretches during use. As understood herein, such slack can lead to kinking of the tubing or excessive wear.

Figure 8 is used to schematically show that at least one angular position sensor 94 can be provided on the motor 58. Without limitation, the angular position sensor may be a Hall effect sensor, or a de stepper motor revolution counter, or a potentiometer type sensor. The sensor 94 generates an output representative of the angular position of the motor. The sensor 94 may be coupled to the motor shaft or the output shaft 60 or other part of the rotating mechanism in the pump.

In any case, the processor 20 shown in Figure 1 can control the motor 58 and can receive the signal from the sensor 94. Using the signal from the sensor 94, the processor 20 can prevent the motor 58 from stopping at an angular position corresponding to at least one roller 46/48 being in a predetermined angular location relative to the raceway 32. In an example, the predetermined location of the roller corresponding to the angular position at which the motor is prevented from stopping is at an arc end 40 or 42 of the raceway 32. This ensures that, particularly when a raceway arc of >180 degrees is used, the rollers will not be in the 12 o'clock and 6 o'clock positions (i.e., adjacent to the ends of the arc), which would interfere with the raceway even when the rotor is in the tube load position and thereby complicate tube loading and unloading.

Thus, the position sensor 94 can be coupled to the motor shaft to indicate critical angular positions to avoid stopping the motor at these positions. The processor 20 can control the motor so that it will not stop on these critical positions. Alternately, the signal from the one or more sensors 94 can be used to indicate non-critical positions, with the processor 20 controlling the motor so it will always stop on these non-critical angular positions. Yet again, a mechanical means, mechanism or other element, e.g., a pin, may be used to ensure that the motor/rotor does not stop in critical positions.

Completing the description, the tube 44 may be configured as a loop as best shown in Figure 6, with the ends of the loop engaged with a manifold 100 in fluid communication with the interior of the manifold 10. In turn, the interior of the manifold 100 may communicate with a cassette 102 such as the cassette shown and described in U.S. Patent 10,792,185.

Such a cassette can be engaged with structure in the control system 14 to exchange heat with working fluid flowing through the cassette 102 and tube 44 and being circulated by the pump 30 shown and described herein to and from a heat exchange member such as the catheter 12 and/or pad 18.

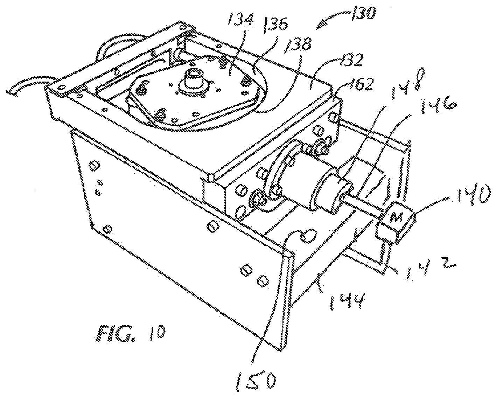

Figure 10 shows an embodiment of the present invention 130 of the peristaltic pump that in all essential respects is identical in configuration and operation to the pump 30 described above with the exceptions noted. Like the pump 30, the pump 130 in Figure 10 includes a rigid, preferably metal or hard plastic raceway 132 and a rotor 134. The raceway 132 or channel may be formed from one or more blocks of material as shown and has an inner arcuate surface 136 which may have a substantially constant radius of curvature. The arcuate surface 136 of Figure 10 is substantially identical in configuration and operation to the arcuate surface 32 in Figure 2. A motor rotates the rotor 134 relative to the raceway 132. As well, like the rotor 32 shown in Figure 2, the rotor 134 shown in Figure 10 may be movable translationally and/or rotationally relative to the raceway 132 between a pump position, in which the rotor 134 is spaced from the midpoint 138 of the inner surface 136 of the raceway 132 a first distance, and a tube load position in which the rotor 134 is spaced from the midpoint 138 a greater, second distance.

However, unlike the pump 30 shown in Figure 2, the pump 130 shown in Figure 10 provides a loading motor 140 to for automated or automatic rotational movement of the rotor relative to the raceway, instead of a manuallymanipulable handle. Thus, the pump 130 in Figure 10 omits, e.g., the handle 70 with hinge mechanism 72.

Instead, the loading motor 140, which may be mounted (e.g., directly on or by means of a bracket 142) to a pump base 144, reciprocatingly drives a push rod 146 to move the rotor 134 between the pump position and tube load position. Advantages associated with using a loading motor to move the rotor include but are not limited to the following: providing convenience for the customer, e.g., the customer may not have to reach back and apply force to move the motor, the customer may not have access to moving parts; minimal or no room for the customer's hand may be needed, which in turn saves space; movement of the rotor may be controlled or allowed only when certain other conditions are met; the rotor may be moved with constant speed; and the force needed to move the rotor may be monitored. The motor 140 may be a direct current (dc) stepper motor or other ac or de motor, or other appropriate motor type and the push rod 146 may be the rack element of a rack-and-pinion gear, with the pinion portion being geared to a shaft rotated by the motor 140. The push rod 146 may extend through a support block 162 that is substantially identical in configuration and operation to the support block 62 shown in Figure 6 to contact and/or be engaged with a motor mount that supports the motor that rotates the rotor 134. One or more radial bearings 148 may be provided as appropriate to radially support elements of the positioning mechanism.

An operating button or key 150 may be manipulable by a person or controller to energize the loading motor 140. The button or key 150 may be positioned on the pump 130 base as shown and may be electrically connected to the controller of the motor 140, with the motor and its controller enclosed in the rectangular box shown at 140. In all other essential respects the pump 130 shown in Figure 10 may be substantially identical in configuration and operation to the pump 30 shown in Figures 2-9.

Components included in one embodiment can be used in other embodiments in any appropriate combination.

"A system having at least one of A, B, and C" (likewise "a system having at least one of A, B. or C" and "a system having at least one of A, B, C") includes systems that have A alone, B alone, C alone, A and B together, A and C together, B and C together, and/or A, B, and C together, etc.

## Claims

1. A pump assembly, comprising:
a raceway (130);
a rotor (134) spaced from the raceway (130);
a rotor motor (58) configured to rotate the rotor (134) to urge fluid through a tube disposed between the raceway (130) and the rotor (134); and
a loading motor (140) coupled to the rotor (134) and configured to move the rotor (134) rotationally relative to the raceway (130), wherein the loading motor (140) is energizable to move the rotor (134) between the pump position and the tube load position.

2. The pump assembly of claim 1, comprising a motor mount (56) supporting the rotor motor (58),
wherein the loading motor (140) is coupled to the motor mount (56) to move the motor mount and thereby move the rotor (134) rotationally relative to the raceway (130) such that motor mount (56) is parallel to a plane of the bottom surface of the raceway (32, 130) when the rotor (34, 134) is in a pump position, and wherein the motor mount (56) is angled (90) relative to the plane of the bottom surface of the raceway when the rotor is in a tube load position.

3. The pump assembly of any preceding claim, wherein the loading motor is configured to reciprocatingly drive a push rod (146) to move the rotor (134) between a pump position and a tube load position.

4. The pump assembly of claim 3, wherein the push rod (146) is the rack element of a rack-and-pinion gear, with the pinion portion being geared to a shaft rotatable by the motor (140).

5. The pump assembly of any preceding claim, wherein the loading motor (140) is configured to move the rotor (134) translationally and rotationally relative to the raceway (130).

6. The pump assembly of any preceding claim, comprising an operating element (150) manipulable by a person or controller to energize the loading motor (140).

7. The pump assembly of any preceding claim, wherein the raceway (130) has an inner surface (136) extending through an arc of at least one hundred eighty degrees (180°), the arc defining a midpoint (138), and the rotor (134) faces the inner surface (136) of the raceway (130), wherein the rotor (134) is spaced from the midpoint a first distance in the pump position, and wherein the rotor (134) is spaced from the midpoint a second distance greater than the first distance in the tube load position.

8. The pump assembly of any preceding claim, wherein the inner surface (136) of the raceway (130) extends through an arc of at least one hundred eighty degrees (180°).

9. The pump assembly of any preceding claim, comprising:
at least one drive roller (46) on the rotor (134), the drive roller having a cylindrical outer surface, at least a portion of the outer surface being configured to urge against a tube disposed between the rotor (134) and the raceway (13), the drive roller having no flanges with peripheries extending beyond the cylindrical outer surface; and
at least one guide roller (48) on the rotor (134), the guide roller having a cylindrical outer surface and top and bottom flanges defining respective peripheries extending beyond the cylindrical outer surface of the guide roller such that a tube disposed between the rotor (134) and the raceway (13) is receivable on the cylindrical outer surface of the guide roller between the flanges.

10. The pump assembly of any preceding claim, comprising plural drive rollers (46) and plural guide rollers (48) on the rotor (134).

11. The pump assembly of any of claims 1 to 4, wherein:
the raceway is an arcuate raceway (32/130) having a concave inner surface (36/136) extending through an arc of at least one hundred eighty degrees (180°), the arc defining a midpoint (38/138);
the rotor (34/134) faces the inner surface (36/136) of the raceway (32/130), the rotor (34/134) being rotatable relative to the raceway (32/130), the rotor (34/134) being mounted relative to the raceway (32/130) between a pump position, wherein the rotor (34/134) is spaced from the midpoint (38/138) a first distance, and a tube load position, wherein the rotor (34/134) is spaced from the midpoint (38/138) a second distance greater than the first distance; and
plural rollers (46, 48) arranged on the rotor (34/134) to contact tubing disposed between the rotor (34/134) and the raceway (32/130) at least when the rotor (34/134) is in the pump position, wherein the motor (58) is prevented from stopping at at least one angular position in which at least one roller (46/48) is in a predetermined location, and preferably, wherein the concave inner surface (36/136) of the raceway (32/130) extends through an arc of between one hundred eighty degrees (180°) and two hundred degrees (200°).

12. The pump assembly of claim 11, wherein the rollers comprise:
at least one drive roller (46) on the rotor (34/134), the drive roller (46) having a cylindrical outer surface, the entire outer surface being configured to urge against a tube (44) disposed between the rotor (34/134) and the raceway (32/130) when the rotor (34/134) is in the pump position and is rotated, the drive roller (46) having no flanges with peripheries extending beyond the cylindrical outer surface; and
at least one guide roller (48) on the rotor (34/134), the guide roller (48) having a cylindrical outer surface and top and bottom flanges (50, 52) defining respective peripheries extending beyond the cylindrical outer surface of the guide roller (48) such that a tube (44) disposed between the rotor (34/134) and the raceway (32/130) is received on the cylindrical outer surface of the guide roller (48) between the flanges (50, 52) when the rotor (34/134) is in the pump position and is rotated.

13. A heat exchange system (10) for patient temperature control, the heat exchange system comprising:
a control system (14); and
a closed loop for working fluid, the closed loop comprising:
a cassette (102) engageable with the control system (14) to exchange heat with the working fluid;
a tube (44) configured as a loop in fluid communication with the cassette; and
an intravascular heat exchange catheter (12) configured to be controlled by the control system (14) to control the patient temperature;
wherein the control system comprises the pump assembly of any preceding claim,
wherein the pump assembly is configured to engage the tube (44) to urge the working fluid through the closed loop through peristalsis.

## Patentansprüche

1. Pumpenbaugruppe, umfassend:
eine Laufbahn (130);
einen Rotor (134), der von der Laufbahn (130) beabstandet ist;
einen Rotormotor (58), der konfiguriert ist, um den Rotor (134) zu drehen, um Fluid durch einen Schlauch zu drücken, das zwischen der Laufbahn (130) und dem Rotor (134) angeordnet ist; und
einen Lademotor (140), der mit dem Rotor (134) gekoppelt und konfiguriert ist, um den Rotor (134) relativ zur Laufbahn (130) in Drehung zu versetzen, wobei dem Lademotor (140) Energie zugeführt werden kann, um den Rotor (134) zwischen der Pumpenposition und der Schlauchlastposition zu bewegen.

2. Pumpenbaugruppe nach Anspruch 1, umfassend eine Motorbefestigung (56), die den Rotormotor (58) trägt,
wobei der Lademotor (140) mit der Motorbefestigung (56) gekoppelt ist, um die Motorbefestigung zu bewegen und dadurch den Rotor (134) relativ zur Laufbahn (130) drehend zu bewegen, dass die Motorbefestigung (56) parallel zu einer Ebene der Bodenfläche der Laufbahn (32, 130) ist, wenn sich der Rotor (34, 134) in einer Pumpposition befindet, und wobei die Motorbefestigung (56) relativ zu der Ebene der Bodenfläche der Laufbahn abgewinkelt (90) ist, wenn sich der Rotor in einer Schlauchlastposition befindet.

3. Pumpenbaugruppe nach einem der vorstehenden Ansprüche, wobei der Lademotor konfiguriert ist, um eine Schubstange (146) hin- und herzubewegen, um den Rotor (134) zwischen einer Pumpenposition und einer Schlauchlastposition zu bewegen.

4. Pumpenbaugruppe nach Anspruch 3, bei der die Schubstange (146) das Zahnstangenelement eines Zahnstangen-Ritzel-Getriebes ist, wobei der Ritzelabschnitt mit einer durch den Motor (140) drehbaren Welle verzahnt ist.

5. Pumpenbaugruppe nach einem der vorstehenden Ansprüche, wobei der Lademotor (140) konfiguriert ist, um den Rotor (134) translatorisch und rotatorisch relativ zu der Laufbahn (130) zu bewegen.

6. Pumpenbaugruppe nach einem der vorstehenden Ansprüche, umfassend ein Bedienelement (150), das von einer Person oder einem Steuergerät betätigt werden kann, um dem Lademotor (140) Energie zuzuführen.

7. Pumpenbaugruppe nach einem der vorstehenden Ansprüche, wobei die Laufbahn (130) eine Innenfläche (136) aufweist, die sich über einen Bogen von mindestens einhundertachtzig Grad (180°) erstreckt, wobei der Bogen einen Mittelpunkt (138) definiert, und der Rotor (134) der Innenfläche (136) der Laufbahn (130) zugewandt ist, wobei der Rotor (134) in der Pumpenposition um einen ersten Abstand vom Mittelpunkt beabstandet ist, und wobei der Rotor (134) in der Schlauchlastposition um einen zweiten Abstand, der größer als der erste Abstand ist, vom Mittelpunkt beabstandet ist.

8. Pumpenbaugruppe nach einem der vorstehenden Ansprüche, wobei sich die Innenfläche (136) der Laufbahn (130) über einen Bogen von mindestens einhundertachtzig Grad (180°) erstreckt.

9. Pumpenbaugruppe nach einem der vorstehenden Ansprüche, umfassend:
mindestens eine Antriebsrolle (46) auf dem Rotor (134), wobei die Antriebsrolle eine zylindrische Außenfläche aufweist, wobei zumindest ein Abschnitt der Außenfläche konfiguriert ist, um gegen einen zwischen dem Rotor (134) und der Laufbahn (13) angeordneten Schlauch zu drücken, wobei die Antriebsrolle keine Flansche mit sich über die zylindrische Außenfläche hinaus erstreckenden Umfänge aufweist; und
mindestens eine Führungsrolle (48) auf dem Rotor (134), wobei die Führungsrolle eine zylindrische Außenfläche und einen oberen und einen unteren Flansch aufweist, die entsprechende Umfänge definieren, die sich über die zylindrische Außenfläche der Führungsrolle hinaus erstrecken, so dass ein zwischen dem Rotor (134) und der Laufbahn (13) angeordneter Schlauch auf der zylindrischen Außenfläche der Führungsrolle zwischen den Flanschen aufgenommen werden kann.

10. Pumpenbaugruppe nach einem der vorstehenden Ansprüche, umfassend mehrere Antriebsrollen (46) und mehrere Führungsrollen (48) auf dem Rotor (134).

11. Pumpenbaugruppe nach einem der Ansprüche 1 bis 4, wobei:
die Laufbahn eine bogenförmige Laufbahn (32/130) mit einer konkaven Innenfläche (36/136) ist, die sich über einen Bogen von mindestens einhundertachtzig Grad (180°) erstreckt, wobei der Bogen einen Mittelpunkt (38/138) definiert;
der Rotor (34/134) der Innenfläche (36/136) der Laufbahn (32/130) zugewandt ist, wobei der Rotor (34/134) relativ zu der Laufbahn (32/130) drehbar ist, wobei der Rotor (34/134) relativ zu der Laufbahn (32/130) zwischen einer Pumpenposition, in der der Rotor (34/134) von dem Mittelpunkt (38/138) um einen ersten Abstand beabstandet ist, und einer Schlauchlastposition, in der der Rotor (34/134) von dem Mittelpunkt (38/138) um einen zweiten Abstand, der größer als der erste Abstand ist, beabstandet ist, montiert ist; und
mehrere Rollen (46, 48), die auf dem Rotor (34/134) angeordnet sind, um die zwischen dem Rotor (34/134) und der Laufbahn (32/130) angeordneten Schläuche zumindest dann zu berühren, wenn sich der Rotor (34/134) in der Pumpstellung befindet, wobei der Motor (58) daran gehindert wird, in mindestens einer Winkelposition anzuhalten, in der sich mindestens eine Rolle (46/48) an einer vorbestimmten Stelle befindet, und wobei sich die konkave Innenfläche (36/136) der Laufbahn (32/130) vorzugsweise über einen Bogen zwischen einhundertachtzig Grad (180°) und zweihundert Grad (200°) erstreckt.

12. Pumpenbaugruppe nach Anspruch 11, wobei die Rollen umfassen:
mindestens eine Antriebsrolle (46) auf dem Rotor (34/134), wobei die Antriebsrolle (46) eine zylindrische Außenfläche aufweist, wobei die gesamte Außenfläche konfiguriert ist, um gegen einen Schlauch (44) zu drücken, der zwischen dem Rotor (34/134) und der Laufbahn (32/130) angeordnet ist, wenn sich der Rotor (34/134) in der Pumpenposition befindet und gedreht wird, wobei die Antriebsrolle (46) keine Flansche mit Umfängen aufweist, die sich über die zylindrische Außenfläche hinaus erstrecken; und
mindestens eine Führungsrolle (48) auf dem Rotor (34/134), wobei die Führungsrolle (48) eine zylindrische Außenfläche und obere und untere Flansche (50, 52) aufweist, die jeweilige Umfänge definieren, die sich über die zylindrische Außenfläche der Führungsrolle (48) hinaus erstrecken, so dass ein zwischen dem Rotor (34/134) und der Laufbahn (32/130) angeordneten Schlauch (44) auf der zylindrischen Außenfläche der Führungsrolle (48) zwischen den Flanschen (50, 52) aufgenommen wird, wenn sich der Rotor (34/134) in der Pumpenposition befindet und gedreht wird.

13. Wärmeaustauschsystem (10) zur Regelung der Patiententemperatur, das Wärmeaustauschsystem umfassend:
ein Steuersystem (14); und
einen geschlossenen Kreislauf für Arbeitsfluid, der Folgendes umfasst:
eine Kassette (102), die mit dem Steuersystem (14) in Eingriff gebracht werden kann, um Wärme mit dem Arbeitsfluid auszutauschen;
einen Schlauch (44), der als Schleife ausgebildet ist und in Fluidverbindung mit der Kassette steht; und
einen intravaskulären Wärmeaustauschkatheter (12), der konfiguriert ist, dass er durch das Steuersystem (14) gesteuert wird, um die Patiententemperatur zu regeln;
wobei das Steuersystem die Pumpenbaugruppe nach einem der vorstehenden Ansprüche umfasst,
wobei die Pumpenbaugruppe konfiguriert ist, dass sie in den Schlauch (44) eingreift, um das Arbeitsfluid durch Peristaltik durch den geschlossenen Kreislauf zu drücken.

## Revendications

1. Ensemble de pompe (10), comprenant :
un chemin de roulement (130) ;
un rotor (134) espacé du chemin de roulement (130) ;
un moteur de rotor (58) configuré pour faire tourner le rotor (134) afin de pousser le fluide à travers un tube agencé entre le chemin de roulement (130) et le rotor (134) ; et
un moteur de chargement (140) couplé au rotor (134) et configuré pour déplacer le rotor (134) en rotation par rapport au chemin de roulement (130), dans lequel le moteur de chargement (140) pouvant être alimenté pour déplacer le rotor (134) entre la position de la pompe et la position de chargement du tube.

2. Ensemble de pompe selon la revendication 1, comprenant un support de moteur (56) supportant le moteur de rotor (58),
dans lequel le moteur de chargement (140) est couplé au support de moteur (56) pour déplacer le support de moteur et déplacer ainsi le rotor (134) en rotation par rapport au chemin de roulement (130) de telle sorte que le support de moteur (56) soit parallèle à un plan de la surface inférieure du chemin de roulement (32, 130) lorsque le rotor (34, 134) est dans une position de pompe, et dans lequel le support de moteur (56) est incliné (90) par rapport au plan de la surface inférieure du chemin de roulement lorsque le rotor est en position de chargement de tube.

3. Ensemble de pompe selon une quelconque revendication précédente, dans lequel le moteur de chargement est configuré pour entraîner en va-et-vient une tige de poussée (146) pour déplacer le rotor (134) entre une position de pompe et une position de chargement de tube.

4. Ensemble de pompe selon la revendication 3, dans lequel la tige de poussée (146) est l'élément à crémaillère d'un engrenage à crémaillère et pignon, la partie pignon étant engrenée sur un arbre pouvant tourner par le moteur (140).

5. Ensemble de pompe selon une quelconque revendication précédente, dans lequel le moteur de chargement (140) est configuré pour déplacer le rotor (134) en translation et en rotation par rapport au chemin de roulement (130).

6. Ensemble de pompe selon une quelconque revendication précédente, comprenant un élément de fonctionnement (150) manipulé par une personne ou un dispositif de commande pour alimenter le moteur de chargement (140).

7. Ensemble de pompe selon une quelconque revendication précédente, dans lequel le chemin de roulement (130) a une surface interne (136) s'étendant sur un arc d'au moins cent quatre-vingts degrés (180°), l'arc définissant un point médian (138), et le rotor (134) fait face à la surface intérieure (136) du chemin de roulement (130), dans lequel le rotor (134) est espacé du point médian d'une première distance dans la position de pompe, et dans lequel le rotor (134) est espacé du point médian d'une seconde distance supérieure à la première distance en position de chargement du tube.

8. Ensemble de pompe selon une quelconque revendication précédente, dans lequel la surface intérieure (136) du chemin de roulement (130) s'étend sur un arc d'au moins cent quatre-vingts degrés (180°).

9. Ensemble de pompe selon une quelconque revendication précédente, comprenant :
au moins un rouleau d'entraînement (46) sur le rotor (134), le rouleau d'entraînement ayant une surface extérieure cylindrique, au moins une partie de la surface externe étant configurée pour pousser contre un tube agencé entre le rotor (134) et le chemin de roulement (13), le rouleau d'entraînement n'ayant pas de rebords dont les périphéries s'étendent au-delà de la surface extérieure cylindrique ; et
au moins un rouleau de guidage (48) sur le rotor (134), le rouleau de guidage ayant une surface extérieure cylindrique et des rebords supérieurs et inférieurs définissant des périphéries respectives s'étendant au-delà de la surface extérieure cylindrique du rouleau de guidage de telle sorte qu'un tube agencé entre le rotor (134) et le chemin de roulement (13) peut être reçu sur la surface extérieure cylindrique du rouleau de guidage entre les brides.

10. Ensemble de pompe selon une quelconque revendication précédente, comprenant plusieurs rouleaux d'entraînement (46) et plusieurs rouleaux de guidage (48) sur le rotor (134).

11. Ensemble de pompe selon l'une quelconque des revendications 1 à 4, dans lequel :
le chemin de roulement est un chemin de roulement arqué (32/130) ayant une surface intérieure concave (36/136) s'étendant sur un arc d'au moins cent quatre-vingts degrés (180°), l'arc définissant un point médian (38/138) ;
le rotor (34/134) fait face à la surface intérieure (36/136) du chemin de roulement (32/130), le rotor (34/134) pouvant tourner par rapport au chemin de roulement (32/130), le rotor (34/134) étant monté par rapport au chemin de roulement (32/130) entre une position de pompe, dans lequel le rotor (34/134) est espacé du point médian (38/138) d'une première distance, et une position de charge de tube, dans lequel le rotor (34/134) est espacé du point médian (38/138) d'une seconde distance supérieure à la première distance ; et
plusieurs rouleaux (46, 48) agencés sur le rotor (34/134) pour entrer en contact avec un tube agencé entre le rotor (34/134) et le chemin de roulement (32/130) au moins lorsque le rotor (34/134) est dans la pompe position, dans lequel le moteur (58) ne peut pas s'arrêter dans au moins une position angulaire dans laquelle au moins un rouleau (46/48) se trouve dans un emplacement prédéterminé, et de préférence, dans lequel la surface intérieure concave (36/136) du le chemin de roulement (32/130) s'étend sur un arc compris entre cent quatre-vingts degrés (180°) et deux cents degrés (200°).

12. Ensemble de pompe selon la revendication 11, dans lequel les rouleaux comprennent :
au moins un rouleau d'entraînement (46) sur le rotor (34/134), le rouleau d'entraînement (46) ayant une surface extérieure cylindrique, la totalité de la surface extérieure étant configurée pour pousser contre un tube (44) agencé entre le rotor (34/ 134) et le chemin de roulement (32/130) lorsque le rotor (34/134) est en position de pompe et tourne, le rouleau d'entraînement (46) n'ayant pas de rebords dont les périphéries s'étendent au-delà de la surface extérieure cylindrique ; et
au moins un rouleau de guidage (48) sur le rotor (34/134), le rouleau de guidage (48) ayant une surface extérieure cylindrique et des rebords supérieurs et inférieurs (50, 52) définissant des périphéries respectives s'étendant au-delà de la surface extérieure cylindrique du rouleau de guidage (48) de telle sorte qu'un tube (44) agencé entre le rotor (34/134) et le chemin de roulement (32/130) soit reçu sur la surface extérieure cylindrique du rouleau de guidage (48) entre les brides (50, 52) lorsque le rotor (34/134) est en position pompe et tourne.

13. Système d'échange de chaleur (10) pour le contrôle de la température d'un patient, le système d'échange de chaleur comprenant :
un système de commande (14) ; et
une boucle fermée pour le fluide de travail, la boucle fermée comprenant :
une cassette (102) pouvant venir en prise avec le système de commande (14) pour échanger de la chaleur avec le fluide de travail ;
un tube (44) configuré comme une boucle en communication fluidique avec la cassette ; et
un cathéter d'échange de chaleur intravasculaire (12) configuré pour être commandé par le système de commande (14) afin de contrôler la température du patient ;
dans lequel le système de commande comprend l'ensemble de pompe selon une quelconque revendication précédente, dans lequel l'ensemble de pompe est configuré pour venir en prise avec le tube (44) afin de pousser le fluide de travail à travers la boucle fermée par péristaltisme.
